# EUROPÄISCHE PATENTSCHRIFT

(11) **EP 4 110 444 B1**
(45) Veröffentlichungstag und Bekanntmachung des Hinweises auf die Patenterteilung: **18.12.2024**
(21) Anmeldenummer: 21706539.0
(22) Anmeldetag: 17.02.2021
(51) Int. Cl.: A61M 16/04, A61B 17/34, A61M 29/00

(54) **VORRICHTUNG FÜR DIE PERKUTAN DILATATIVE PUNKTIONSTRACHEOTOMIE**
DEVICE FOR PERCUTANEOUS DILATIONAL TRACHEOTOMY
DISPOSITIF POUR TRACHÉOTOMIE PERCUTANÉE PAR DILATATION

(30) Priorität: 26.02.2020 DE 102020105048
(43) Veröffentlichungstag der Anmeldung: 04.01.2023
(73) Patentinhaber: Coloplast A/S, 3050 Humlebaek (DK)
(72) Erfinder: SCHNELL, Ralf, 63500 Seligenstadt (DE)
(86) Internationale Anmeldenummer: PCT/EP2021/053808
(87) Internationale Veröffentlichungsnummer: WO 2021/170457

(56) Entgegenhaltungen:
- EP-A1- 2 301 618
- EP-B1- 2 301 618
- US-A- 5 169 387
- US-A1- 2004 092 879
- US-A1- 2010 300 451
- US-A1- 2018 280 647
- TRACOE MEDICAL: "Quality by People for People", 30 November 2017, PRODUCT CATALOG, TRACOE MEDICAL, DE, PAGE(S) 1 - 111, XP009541538

## Beschreibung

Die vorliegende Erfindung betrifft eine Vorrichtung für die perkutan dilatative Punktionstracheotomie, wobei die Vorrichtung eine Punktionskanüle, einen Führungskatheter und einen Führungsdraht aufweist. Von der vorliegenden Erfindung umfasst ist auch ein Set, dass die erfindungsgemäße Vorrichtung für die perkutan dilatative Punktionstracheotomie enthält sowie einen Dilatator zum Erweitern des Tracheostomas und wahlweise als weitere Komponenten ein Skalpell, einen Prädilatator, eine Einführhilfe für den Führungsdraht, einen Armierungskatheter mit Sicherheitssperre für den Führungsdraht, einen Satz Kompressen und/oder eine Spritze.

Die perkutan dilatative Dilatationstracheotomie ist eine Methode zur Einbringung eines Tracheotomietubus in die Luftröhre eines Patienten. Als Alternative zur chirurgischen Methode wird hier zunächst einmal mit Hilfe eines Over-the-needle-Katheters ein Zugang zur Trachea gelegt. Dies geschieht typischerweise zwischen dem 2. und 3. oder dem 3. und 4. Trachealring.

Beispiele für Vorrichtungen für die perkutan dilatative Punktionstracheotomie sind im europäischen Patent EP 2 301 618 und in der US-Patentanmeldung US 2010/300451 offengelegt.

Die für die perkutan dilatative Dilatationstracheotomie notwendigen Einzelteile werden typischerweise in aufeinander abgestimmten Sets angeboten, die zusätzlich zum Over-the-needle-Katheter auch weitere Komponenten enthalten, die bei dem Eingriff zur Anwendung kommen können, wie z.B. ein Skalpell, einen Prädilatator, einen Dilatator, einen Führungsdraht, eine Einführhilfe für den Führungsdraht, einen Armierungskatheter mit Sicherheitssperre für den Führungsdraht, einen Satz Kompressen und/oder eine Spritze.

Over-the-needle-Katheter bestehen aus einer hohlen angespitzten Punktionskanüle aus Stahl, die von einem eng anliegenden Führungskatheter aus Kunststoff umgeben ist. Sowohl Nadel als auch Katheter haben an dem vom Patienten abgewandten proximalen Ende oft jeweils einen Konnektor, wie z.B. einen weiblichen Luer-Konnektor. Die Punktionskanüle gibt dem Führungskatheter ausreichend Festigkeit, um ihn in den Patienten einbringen zu können.

Nach erfolgter Einbringung des Over-the-needle-Katheters wird die Punktionskanüle aus dem im Patienten verbleibenden Führungskatheter herausgezogen, damit dann ein Führungsdraht, der sogenannte Seldingerdraht, durch den Führungskatheter in die Trachea eingeführt werden kann. Der Führungsdraht dient als Leitschiene während der Dilatation mit dafür vorgesehenen Dilatatoren und während der anschließenden Einführung des Tracheotomietubus. Auf diese Weise wird das Risiko von Trachealwandverletzungen minimiert und sichergestellt, dass Dilatatoren und Tracheotomietubus tatsächlich in die Trachea eingeführt werden und nicht versehentlich in der *Via falsa* landen.

Nachdem die Punktionskanüle aus dem Führungskatheter herausgezogen wurde, besteht das Risiko, dass der im Patienten verbleibende Führungskatheter abknickt. Dies kann v.a. dann leicht passieren, wenn der Anwender nach Entnahme der stabilisierenden Punktionskanüle versucht die Position bzw. den Winkel des Katheters relativ zur Position der Trachea zu verändern. Wenn der Führungskatheter einmal abgeknickt ist, lässt sich der Führungsdraht oft nicht mehr durch den Führungskatheter durchführen, da der Knick eine Engstelle darstellt, durch die sich der Führungsdraht nicht durchschieben lässt.

Diese Situation lässt sich oft auch nicht mehr retten, indem man die bereits entnommene Punktionskanüle wieder einführt, da dies leicht dazu führen kann, dass der Führungskatheter durch die wieder eingeführte Kanüle punktiert wird. In vielen Fällen wird daher der Punktionsprozess noch einmal mit einem neuen Over-the-needle-Katheter wiederholt, und wenn hierfür keine passenden Einzelteile zur Hand sind, wird oft ein neues Tracheotomieset geöffnet, was natürlich mit zusätzlichen Verbrauchskosten verbunden ist.

Um das oben beschriebene Problem zu lösen, wird mit der vorliegenden Erfindung eine Vorrichtung für die perkutan dilatative Punktionstracheotomie vorgeschlagen, wie im unabhängigen Anspruch 1 definiert, die u.a. eine Punktionskanüle, einen Führungskatheter und einen Führungsdraht aufweist, wobei die Punktionskanüle an deren distalem Ende eine geschliffene Spitze aufweist, wobei der maximale Außendurchmesser der Punktionskanüle geringer ist als der minimale Innendurchmesser des Führungskatheters, und wobei die Punktionskanüle so weit in den Führungskatheter einführbar ist, dass die geschliffene Spitze der Punktionskanüle über das distale Ende des Führungskatheters hinausragt, wobei der maximale Durchmesser des Führungsdrahts geringer ist als der minimale Innendurchmesser der Punktionskanüle, sodass der Führungsdraht so durch die Punktionskanüle hindurchführbar ist, dass dessen Ende über die geschliffene Spitze der Punktionskanüle hinausragt.

Der Vorteil der vorliegenden Erfindung besteht darin, dass die Querschnitte von Punktionskanüle, Führungskatheter und Führungsdraht so dimensioniert sind, dass der Führungsdraht durch die in dem Führungskatheter angeordnete Punktionskanüle durchgeführt werden kann. Es ist daher nicht mehr notwendig, nach erfolgter Einbringung des Führungskatheters in die Trachea die Punktionskanüle herauszuziehen, um den Führungsdraht einführen zu können. Es besteht somit bei Verwendung der erfindungsgemäßen Vorrichtung auch nicht die Gefahr, dass der Katheter abknickt, da dieser auch während des Einführens des Führungsdrahts von der Punktionskanüle stabilisiert wird.

Mit herkömmlichen Tracheotomievorrichtungen ist dieser Vorteil nicht zu erreichen, da bei diesen der Innendurchmesser des Führungskatheters gerade groß genug ist, damit sich der Führungsdraht hindurchführen lässt. Andererseits ist bei herkömmlichen Tracheotomievorrichtungen der Innendurchmesser des Führungskatheters so gering wie möglich, um insgesamt bei der Punktion einen möglichst geringen Querschnitt zu haben.

Aus den vorgenannten Gründen ist bei herkömmlichen Tracheotomievorrichtungen der Innendurchmesser des Führungskatheters etwa 0,1 bis 0,4 mm größer als der Außendurchmesser des Führungsdrahtes. Dies führt dazu, dass bei herkömmlichen Tracheotomievorrichtungen der Außendurchmesser des Führungsdrahtes größer ist als der Innendurchmesser der Punktionskanüle. Es ist bei herkömmlichen Tracheotomievorrichtungen daher nicht möglich, den Führungsdraht durch die Punktionskanüle zu schieben.

Im Gegensatz hierzu sind bei der vorliegenden Erfindung Führungskatheter und Punktionskanüle so dimensioniert, dass der Führungsdraht durch die Punktionskanüle geführt werden kann. Bei manchen Ausführungsformen der Erfindung weist der Führungsdraht hierfür einen maximalen Durchmesser im Bereich von 1,0 bis 1,5 mm, vorzugsweise im Bereich von 1,2 bis 1,3 mm, auf. Bei bestimmten Ausführungsformen ist der minimale Innendurchmesser der Punktionskanüle 0,1 bis 0,4 mm größer als der maximale Durchmesser des Führungsdrahts. Bei spezifischen Ausführungsformen ist der minimale Innendurchmesser der Punktionskanüle 0,2 bis 0,4 mm größer als der maximale Durchmesser des Führungsdrahts. Der minimale Innendurchmesser des Führungskatheters ist bei bestimmten Ausführungsformen der Erfindung 0,05 bis 0,3 mm größer als der maximale Außendurchmesser der Punktionskanüle. Bei spezifischen Ausführungsformen ist der minimale Innendurchmesser des Führungskatheters 0,05 bis 0,15 mm größer als der maximale Durchmesser der Punktionskanüle.

Die Punktionskanüle besteht aus einer Hohlnadel, die an deren distalem Ende eine geschliffene Spitze aufweist. Vorzugsweise ist am proximalen Ende der Hohlnadel ein Konnektor angeordnet. Der Führungskatheter besteht aus einer Führungsröhre, die an deren distalem Ende vorzugsweise konisch zulaufend ausgeführt ist. Auch am proximalen Ende der Führungsröhre ist optional ein Konnektor angeordnet. Vorhandene Konnektoren sind zum Verbinden mit einem herkömmlichen medizinischen Schlauchsystem geeignet (z.B. weiblicher Luer-Konnektor).

Die Begriffe "distal" und "proximal" werden im Sinne der vorliegenden Erfindung aus der Sicht eines die Tracheotomievorrichtung anwendenden Arztes verwendet, d.h. das proximale Ende der Punktionskanüle ist das Ende, welches nach der Punktion außerhalb des Patientenkörpers verbleibt, während das distale Ende bei der Punktion durch die Haut in die Trachea des Patienten eingeführt wird.

Vorzugsweise ragt die geschliffene Spitze der Punktionskanüle 1 bis 10 mm, besonders bevorzugt 2 bis 6 mm, über das distale Ende des Führungskatheters hinaus. Hierfür sind die Längen der Hohlnadel und der Katheterröhre sowie gegebenenfalls die Position und Gestalt der Konnektoren an Punktionskanüle und Führungskatheter entsprechend gewählt.

Vorzugsweise hat die Hohlnadel der Punktionskanüle eine Länge im Bereich von 4 cm bis 9 cm. Der maximale Außendurchmesser der Hohlnadel liegt vorzugsweise im Bereich von 1,3 bis 2,1 mm. Bei bestimmten Ausführungsformen liegt der maximale Außendurchmesser der Hohlnadel im Bereich von 1,5 bis 2,0 mm. Der minimale Innendurchmesser der Hohlnadel liegt vorzugsweise im Bereich von 1,1 bis 1,9 mm. Bei bestimmten Ausführungsformen liegt der minimale Innendurchmesser der Hohlnadel im Bereich von 1,2 bis 1,8 mm. Die Wandstärke der Hohlnadel liegt vorzugsweise im Bereich von 0,1 bis 0,2 mm.

Vorzugsweise hat die Katheterröhre des Führungskatheters eine Länge im Bereich von 3 cm bis 8 cm. Der maximale Außendurchmesser der Katheterröhre liegt vorzugsweise im Bereich von 1,5 bis 2,5 mm. Bei bestimmten Ausführungsformen liegt der maximale Außendurchmesser der Katheterröhre im Bereich von 1,6 bis 2,3 mm. Der minimale Innendurchmesser der Katheterröhre liegt vorzugsweise im Bereich von 1,4 bis 2,1 mm. Bei bestimmten Ausführungsformen liegt der minimale Innendurchmesser der Katheterröhre im Bereich von 1,6 bis 1,9 mm. Die Wandstärke der Katheterröhre des Führungskatheters liegt vorzugsweise im Bereich von 0,1 bis 0,2 mm.

Die Hohlnadel der Punktionskanüle ist bei bevorzugten Ausführungsformen aus Metall, besonders bevorzugt aus Stahl. Die Führungsröhre des Führungskatheters besteht vorzugsweise aus Kunststoff bevorzugt FEP (Perfluorethylenpropylen). Vorhandene Konnektoren an der Punktionskanüle und/oder dem Führungskatheter sind ebenfalls vorzugsweise aus Kunststoff. Bei besonders bevorzugten Ausführungsformen sind die Führungsröhre und ein daran angeordneter Konnektor einstückig aus einem Kunststoffmaterial geformt.

Der Kunststoff der Führungsröhre kann entweder aus einem transparenten oder nicht-transparenten Kunststoffmaterial bestehen, wahlweise mit einem oder mehreren weißen oder farbigen Längsstreifen zur Erleichterung der endoskopischen Lagekontrolle. Bei bestimmten Ausführungsformen enthält das Kunststoffmaterial der Führungsröhre ein Röntgenkontrastmittel (z.B. Bariumsulfat).

Bei bestimmten Ausführungsformen, bei denen am proximalen Ende von Punktionskanüle und Führungskatheter Konnektoren angeordnet sind, ist der Konnektor an der Punktionskanüle in dem Konnektor an dem Führungskatheter so in einer ersten stabilen Einschubposition angeordnet, dass die geschliffene Spitze der Punktionskanüle 1 bis 10 mm, besonders bevorzugt 2 bis 6 mm, über das distale Ende des Führungskatheters hinausragt. Vorzugsweise kann bei diesen Ausführungsformen durch Ziehen oder Drehen an dem Konnektor der Punktionskanüle diese in eine zweite stabile Einschubposition gebracht werden, in der die geschliffene Spitze nicht mehr über den Führungskatheter hinausragt.

Um die Punktionskanüle durch Drehen in eine zweite stabile Einschubposition zu bringen, in der die geschliffene Spitze nicht über den Führungskatheter hinausragt, ist bei manchen Ausführungsformen an der Innenwand des Führungskatheter-Konnektors ein Innen- oder Außengewinde angeordnet und an der Außenwand des Punktionskanülen-Konnektors ein entsprechendes Außen- oder Innengewinde. Alternativ ist auf der Innenwand des Führungskatheter-Konnektors oder an der Außenwand des Punktionskanülen-Konnektors ein Innengewinde angeordnet und auf der jeweiligen anderen Wand (Außenwand des Führungskatheter-Konnektors bzw. Innenwand des Punktionskanülen-Konnektors) ein vorstehender Steg oder eine vorstehende Nase, der bzw. die mit dem auf der anderen Wand befindlichen Gewinde in Eingriff treten kann.

Vorzugsweise definiert die aufeinander abgestimmte Geometrie des/der Gewinde(s) bzw. Stege/Nasen wenigstens zwei unterschiedliche Einrastpositionen, wobei in einer ersten Einrastposition die geschliffene Spitze der Punktionskanüle 1 bis 10 mm, besonders bevorzugt 2 bis 6 mm, über das distale Ende des Führungskatheters hinausragend stabil gehalten wird, und wobei in einer zweiten Einrastposition die geschliffene Spitze der Punktionskanüle nicht über den Führungskatheter hinausragt.

Der Führungsdraht besteht vorzugsweis aus Metall oder Kunststoff. Ein bevorzugtes Metall des Führungsdrahtes ist Stahl, Nitinol oder eine Kombination hiervon. Die Ausführungsformen mit Metalldraht weisen vorzugsweise eine Kunststoffbeschichtung auf dem Metalldraht auf. Bei bestimmten Ausführungsformen handelt es sich bei dem Material der Kunststoffbeschichtung um Teflon (Polytetrafluorethylen) oder FEP (Perfluorethylenpropylen). Bestimmte Ausführungsformen sind dadurch gekennzeichnet, dass das Ende des Führungsdrahtes, dass im durch die Punktionskanüle hindurchgeführten Zustand über das Ende der Punktionskanüle hinausragt, viertel- bis dreiviertelkreisförmig gebogen ist. Diese Art von Führungsdraht wird häufig als Führungsdraht bezeichnet.

Um den Führungsdraht leichter einführen zu können, weitet sich bei der ersten der drei alternativen Möglichkeiten der in Anspruch 1 beanspruchten Erfindung das Lumen der Hohlnadel der Punktionskanüle an dem der geschliffenen Spitze gegenüberliegenden Ende der Punktionskanüle in proximaler Richtung trichterförmig.

Bei der zweiten alternativen Möglichkeit der in Anspruch 1 beanspruchten Erfindung mit einem Konnektor am proximalen Ende der Punktionskanüle verjüngt sich vorzugsweise das Lumen des Konnektors trichterförmig in Richtung des Lumens der Punktionskanüle, damit der Führungsdraht leichter in die Hohlnadel eingeführt werden kann. Besonders bevorzugt schließt die Innenwand des Konnektors am proximalen Ende der Hohlnadel mit der Hohlnadelöffnung ab.

Bei der dritten alternativen Möglichkeit der in Anspruch 1 beanspruchten Erfindung, bei der das Lumen eines Konnektors am proximalen Ende der Punktionskanüle im wesentlichen zylinderförmig ist, ist vorzugsweise ein Adapter passgenau im Lumen des Konnektors angeordnet, wobei der Adapter selbst ein Lumen aufweist, das sich trichterförmig in Richtung des Lumens der Punktionskanüle verjüngt, damit der Führungsdraht leichter in die Hohlnadel eingeführt werden kann. Besonders bevorzugt schließt die Innenwand des in den Konnektor eingesetzten Adapters am proximalen Ende der Hohlnadel mit der Hohlnadelöffnung bündig ab.

Die vorliegende Erfindung betrifft eine Vorrichtung für die perkutan dilatative Punktionstracheotomie, wobei die Vorrichtung eine Punktionskanüle, einen Führungskatheter und einen Führungsdraht aufweist. Bei bestimmten Ausführungsformen ist diese Vorrichtung Teil eines Sets für die perkutan dilatative Punktionstracheotomie, das darüber hinaus auch einen Dilatator zum Erweitern des Tracheostomas aufweist. Ein solcher Dilatator weist einen Dilatationsbereich mit in Längsrichtung kontinuierlich zunehmendem Durchmesser auf, und bei bestimmten Ausführungsformen ist im Dilatator in Längsrichtung ein Führungskanal vorgesehen, dessen minimaler Innendurchmesser größer ist als der maximale Durchmesser des Führungsdrahts, sodass der Führungsdraht durch den Führungskanal hindurchführbar ist.

Im Dilatationsbereich nimmt der äußere Durchmesser des Dilatators vorzugweise auf bis zu 1,2 bis 1,5 cm zu. Bei bevorzugten Ausführungsformen liegt der minimale Innendurchmesser des Führungskanals des Dilatators im Bereich von 1,0 bis 3,0 mm.

Bei bestimmten Ausführungsformen des erfindungsgemäßen Sets enthält dieses zusätzlich ein Skalpell, einen Prädilatator, eine Einführhilfe für den Führungsdraht, einen Satz Kompressen, einen Armierungskatheter mit Sicherheitssperre für den Führungsdraht und/oder eine Spritze, sowie gegebenenfalls ein Tracheotomietubus und oder Einführhilfen zur Einführung eines Tracheotomietubus.

Bei den Ausführungsformen des Sets mit Prädilatator zum Erweitern des Tracheostomas hat dieser Prädilatator einen äußeren Durchmesser, der größer ist als der äußere Durchmesser des Führungskatheters und der kleiner ist als der mittlere äußere Durchmesser des Dilatators, wobei im Prädilatator in Längsrichtung ein Führungskanal vorgesehen ist, dessen minimaler Innendurchmesser größer ist als der maximale Durchmesser des Führungsdrahts, sodass der Führungsdraht durch den Führungskanal hindurchführbar ist, wobei der minimale Innendurchmesser des Führungskanals des Prädilatators 0,1 bis 1,0 mm größer ist als der maximale Durchmesser des Führungsdrahts.

Bei den Ausführungsformen des Sets mit Armierungskatheter hat dieser einen äußeren Durchmesser, der geringer ist als der minimale innere Durchmesser des Dilatators, damit der Armierungskatheter durch den Führungskanal des Dilatators geführt werden kann. Der minimale Innendurchmesser des Armierungskatheters ist größer als der maximale Durchmesser des Führungsdrahts, sodass der Führungsdraht durch den Armierungskatheter hindurchführbar ist, wobei der minimale Innendurchmesser des Armierungskatheters 0,1 bis 1,0 mm größer ist als der maximale Durchmesser des Führungsdrahts.

Bei den Ausführungsformen des Sets mit Einführhilfe für den Führungsdraht, ist die Einführhilfe für den Führungsdraht vorzugsweise an dem Ende des Führungsdrahtes, das im durch die Punktionskanüle hindurchgeführten Zustand über das Ende der Punktionskanüle hinausragt, angeordnet, wobei die Einführhilfe in Längsrichtung und zentral einen Führungskanal aufweist, dessen minimaler Innendurchmesser größer ist als der maximale Durchmesser des Führungsdrahts, sodass der Führungsdraht durch den zentralen Führungskanal hindurchführbar ist, wobei ein Ende des Führungskanals in einem sich konisch verjüngenden Abschnitt der Einführhilfe mündet. Dieser sich konisch verjüngende Abschnitt der Einführhilfe ist in Größe und Gestalt so ausgeführt, dass dieser Abschnitt in das proximale Ende der Punktionskanüle sei es mit oder ohne Konnektor oder mit oder ohne Adapter eingeführt werden kann, um den Führungsdraht leichter in die Hohlnadel einführen zu können.

Für Zwecke der ursprünglichen Offenbarung wird darauf hingewiesen, dass sämtliche Merkmale, wie sie sich aus der vorliegenden Beschreibung, den Zeichnungen und den Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder technische Gegebenheiten derartige Kombinationen unmöglich oder sinnlos machen. Auf die umfassende, explizite Darstellung sämtlicher denkbarer Merkmalskombinationen wird hier nur der Kürze und der Lesbarkeit der Beschreibung wegen verzichtet.

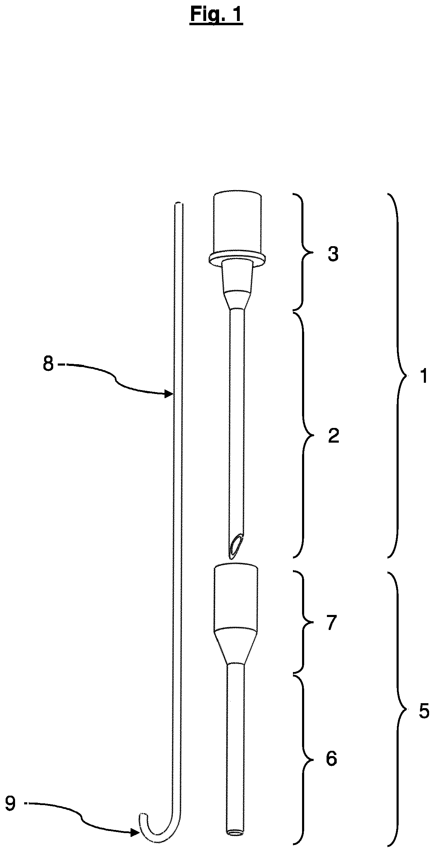

Einzelne Beispiele der oben erwähnten spezifischen Ausführungsformen der Erfindung werden anhand der anhängenden Figuren 1 bis 5 angegeben, wobei
- Figur 1: eine Draufsicht auf eine Ausführungsform einer erfindungsgemäßen Vorrichtung für die perkutan dilatative Punktionstracheotomie zeigt, wobei die Vorrichtung eine Punktionskanüle, einen Führungskatheter und einen Führungsdraht mit gebogenem Ende aufweist, und wobei diese drei Bestandteile separat, d.h. nicht ineinander gesteckt dargestellt sind,

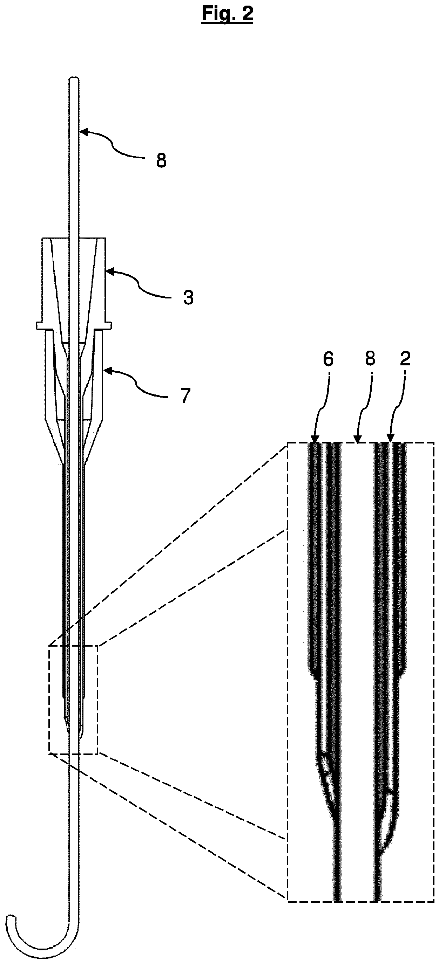

- Figur 2: die Ausführungsform der Erfindung gemäß Figur 1 im Querschnitt zeigt, wobei die Punktionskanüle, der Führungskatheter und der Führungsdraht ineinander gesteckt dargestellt sind,

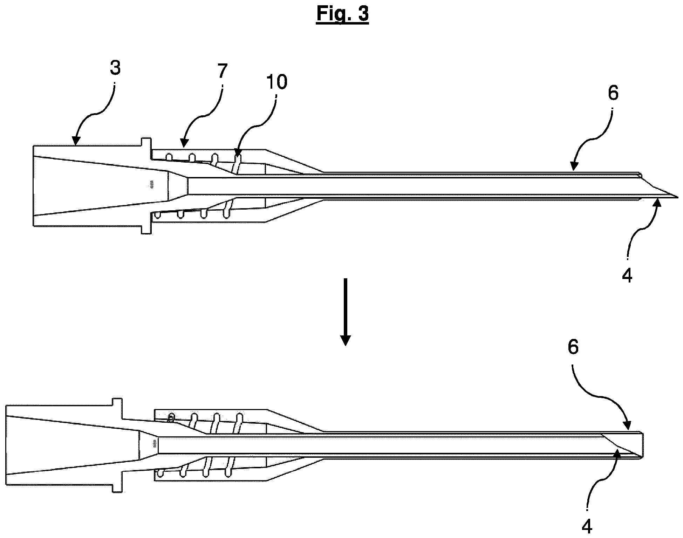

- Figur 3: die Punktionskanüle und den Führungskatheter einer Ausführungsform der Erfindung im Querschnitt zeigt, wobei die Punktionskanüle in zwei unterschiedlichen Positionen in den Führungskatheter eingesteckt dargestellt ist,

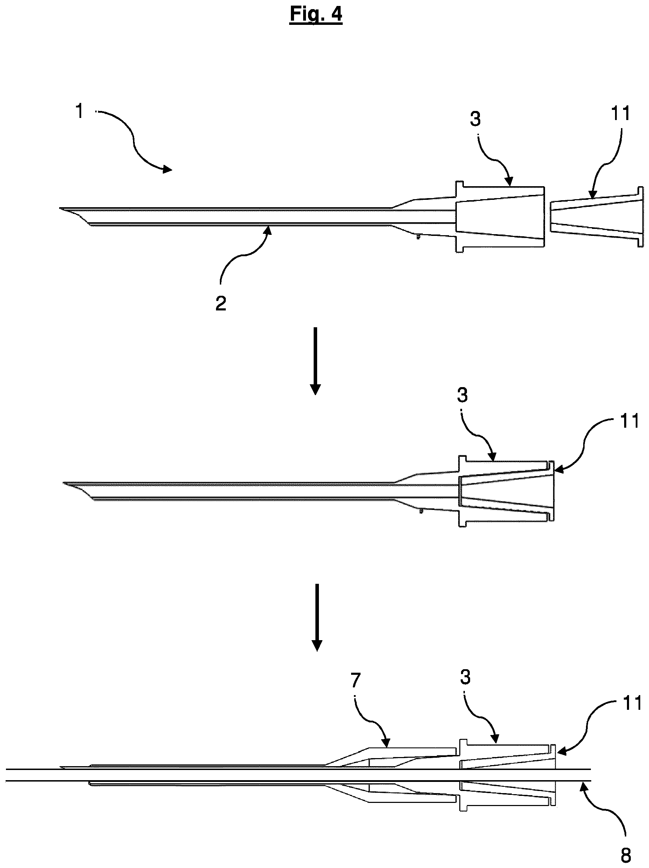

- Figur 4: einen Querschnitt durch eine Punktionskanüle einer Ausführungsform der Erfindung in drei unterschiedlichen Zuständen zeigt, wobei die ersten beiden Zustände illustrieren, wie in das Lumen des am proximalen Ende der Punktionskanüle angeordneten Konnektors ein Adapter eingesteckt wird und im dritten Zustand die Punktionskanüle mit durch die Punktionskanüle geführtem Führungsdraht und in den Führungskatheter eingesteckt dargestellt ist, und

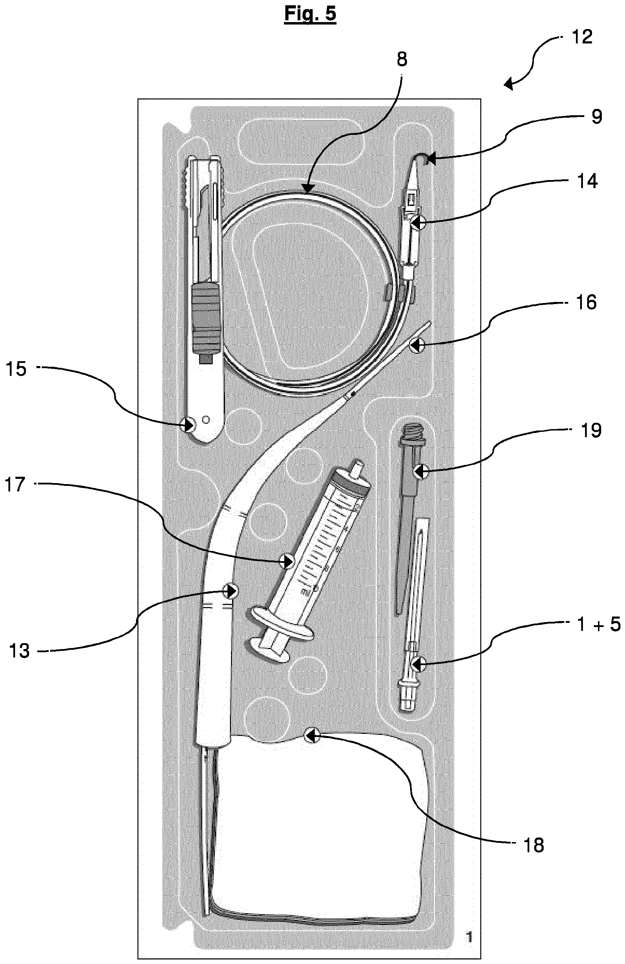

- Figur 5: eine Draufsicht auf eine Ausführungsform eines Sets für die perkutan dilatative Punktionstracheotomie, wobei das Set eine Punktionskanüle, einen Führungskatheter und einen Führungsdraht sowie einen Dilatator, ein Skalpell, einen Prädilatator, eine Einführhilfe für den Führungsdraht, einen Armierungskatheter mit Sicherheitssperre für den Führungsdraht, einen Satz Kompressen und eine Spritze umfasst.

Im Einzelnen sind in den oben erwähnten Figuren die folgenden Merkmale bzw. Merkmalskombinationen dargestellt.

In Figur 1 ist eine Draufsicht auf eine Ausführungsform einer erfindungsgemäßen Vorrichtung für die perkutan dilatative Punktionstracheotomie dargestellt, wobei diese Vorrichtung eine Punktionskanüle 1, einen Führungskatheter 5 und einen Führungsdraht 8 aufweist. In der Darstellung von Figur 1 sind die drei Bestandteile separat, d.h. nicht ineinandergesteckt, abgebildet. In der Darstellung von Figur 1 ist bei der Punktionskanüle 1 der freiliegende Abschnitt der Hohlnadel 2 sowie der am proximalen Ende der Punktionskanüle angeordnete Kanülen-Konnektor 3 zu erkennen. Wie auch bei herkömmlichen Punktionskanülen üblich, wird ein kurzer proximaler Abschnitt der Hohlnadel 2 vom Kanülen-Konnektor 3 umfasst, und durch den hier überlappenden Bereich ist die Hohlnadel 2 mit dem Kanülen-Konnektor 3 fest verbunden.

Auch der hier dargestellte Führungskatheter 5 besteht aus zwei Untereinheiten, nämlich der Katheterröhre 6 und dem Katheter-Konnektor 7. Bei der hier dargestellten Ausführungsform aus Kunststoff sind Katheterröhre und Katheter-Konnektor einstückig aus Kunststoff ausgeführt, sodass sich das Kunststoffmaterial ab dem Übergang von der Katheterröhre 6 zum Katheter-Konnektor 7 konisch aufweitet, um dann in proximaler Richtung in einen zylindrischen Abschnitt überzugehen.

Der Führungsdraht 8 ist hier stark verkürzt dargestellt und weist an seinem distalen Ende ein halbkreisförmig gebogenes Ende auf 9.

Figur 2 zeigt die in Figur 1 in separate Bestandteile zerlegte Ausführungsform der Erfindung im zusammengesetzten Zustand im Querschnitt. Dabei ist gut zu erkennen, wie der Führungsdraht 8 durch die Hohlnadel 2 der Punktionskanüle verläuft und wie die Hohlnadel 2 der Punktionskanüle in der Katheterröhre 6 des Führungskatheters angeordnet ist. Im hier dargestellten zusammengesetzten Zustand ist das distale Ende des Kanülen-Konnektors 3 in das Lumen des Katheter-Konnektor 7 eingeführt. Durch einen am Kanülen-Konnektor vorgesehenen Anschlagpunkt wird eine Position definiert, bei der das distale Ende der Hohlnadel 2 über das distale Ende der Katheterröhre 6 hinausragt.

Die Querschnittsdarstellung einer Ausführungsform der Erfindung, die in Figur 3 dargestellt ist, zeigt zwei diskrete stabile Einschubpositionen für die in den Führungskatheter eingeführte Punktionskanüle. In dem oben in Figur 3 dargestellten Zustand befindet sich die Punktionskanüle in einer ersten stabilen Einschubposition, die wie bei dem oben für Figur 2 beschriebenen Zustand durch einen am Kanülen-Konnektor vorgesehenen Anschlag definiert wird, und in diesem Zustand ragt die am distalen Ende der Hohlnadel vorgesehene geschliffene Spitze 4 über das distale Ende der Katheterröhre 6 hinaus. Im Zustand, der in der unteren Darstellung von Figur 3 abgebildet ist, befindet sich die Punktionskanüle in einer zweiten stabilen Einschubposition, bei der die geschliffene Spitze 4 am distalen Ende der Hohlnadel nicht über das distale Ende der Katheterröhre 6 hinausragt. Diese zweite stabile Einschubposition wird definiert durch das an der Innenwand des Katheter-Konnektors 7 vorgesehene Innengewinde 10. Durch Drehen der Punktionskanüle gegen den Führungskatheter kann die Punktionskanüle also aus der vorgenannten ersten stabilen Einschubpositionen in eine zweite stabile Einschubposition bewegt werden, was dazu führt, dass die geschliffene Spitze 4 am distalen Ende der Punktionskanüle in das Lumen des Führungskatheters zurückgezogen wird.

In Figur 4 ist oben ein Querschnitt durch eine Punktionskanüle einer Ausführungsform der Erfindung dargestellt, bei der die Innenwand des Kanülen-Konnektors 3 in distaler Richtung zum Lumen der Hohlnadel 2 leicht konisch zuläuft. Allerdings verbleibt ein Abschnitt um das proximale Hohlnadelende herum, der senkrecht zur Längsrichtung der Hohlnadel 2 verläuft. Beim Einführen eines Führungsdrahtes kann das Ende des Führungsdrahtes an diesen senkrechten Flächen anstoßen, wodurch das Einführen des Führungsdrahtes in die Hohlnadel stark erschwert wird. Um dies zu vermeiden, ist bei dieser Ausführungsform ein Adapter 11 vorgesehen, der in das Lumen des Kanülen-Konnektors 3 passgenau eingesetzt werden kann (siehe Abbildung in der Mitte), wodurch die oben beschriebenen senkrechten Flächen rechts und links der Hohlnadeleintrittsöffnung eliminiert werden. Hierfür weist der Adapter 11 ein Lumen auf, das sich trichterförmig in Richtung des Lumens der Punktionskanüle verjüngt, sodass die Innenwand des Adapters mit der Eintrittsöffnung der Hohlnadel 2 bündig zum Abschluss kommt.

Wird nun ein Führungsdraht 8 in die Punktionskanüle 1 eingeführt, so wird er entlang der Innenwand des Adapters 11 direkt in die Eintrittsöffnung der Hohlnadel 2 geführt, was das Einführen des Führungsdrahtes 8 in die Punktionskanüle 1 deutlich erleichtert.

In Figur 5 ist eine Draufsicht auf eine Ausführungsform eines Sets 12 für die perkutan dilatative Punktionstracheotomie dargestellt, wobei dieses Set 12 neben der Punktionskanüle 1, dem Führungskatheter 5 und dem Führungsdraht 8 zusätzlich noch einen Prädilatator 19, einen Dilatator 13, eine Einführhilfe 14 für den Führungsdraht 8, ein Skalpell 15, einen Armierungskatheter 16 mit Sicherheitssperre für den Führungsdraht 8, eine Spritze 17 und ein Set Kompressen 18 enthält

Die Erfindung ist durch die beigefügten Ansprüche definiert.

### Bezugszeichenliste:

- 1: Punktionskanüle
- 2: Hohlnadel
- 3: Kanülen-Konnektor
- 4: geschliffene Spitze
- 5: Führungskatheter
- 6: Katheterröhre
- 7: Katheter-Konnektor
- 8: Führungsdraht
- 9: gebogenes Ende
- 10: Gewinde
- 11: Adapter
- 12: Tracheotomieset
- 13: Dilatator
- 14: Einführhilfe für Führungsdraht
- 15: Skalpell
- 16: Armierungskatheter mit Sicherheitssperre
- 17: Spritze
- 18: Kompressen
- 19: Prädilatator

## Patentansprüche

1. Vorrichtung für die perkutan dilatative Punktionstracheotomie, wobei die Vorrichtung eine Punktionskanüle (1), einen Führungskatheter (5) und einen Führungsdraht (8) aufweist, wobei die Punktionskanüle an deren distalem Ende eine geschliffene Spitze (4) aufweist, wobei der maximale Außendurchmesser der Punktionskanüle geringer ist als der minimale Innendurchmesser des Führungskatheters, und wobei die Punktionskanüle so weit in den Führungskatheter einführbar ist, dass die geschliffene Spitze der Punktionskanüle über das distale Ende des Führungskatheters hinausragt, wobei der maximale Durchmesser des Führungsdrahts geringer ist als der minimale Innendurchmesser der Punktionskanüle, sodass der Führungsdraht (8) so durch die Punktionskanüle (1) hindurchführbar ist, dass dessen Ende über die geschliffene Spitze (4) der Punktionskanüle hinausragt, **dadurch gekennzeichnet, dass** sich das Lumen der Punktionskanüle an deren proximalem Ende, dass der geschliffenen Spitze gegenüberliegt, trichterförmig weitet oder am proximalen Ende der Punktionskanüle ein Konnektor (3) angeordnet ist, dessen Lumen sich trichterförmig in Richtung des Lumens der Punktionskanüle verjüngt, oder am proximalen Ende der Punktionskanüle ein Adapter (11) angeordnet ist, desser Lumen sich trichterförmig in Richtung des Lumens der Punktionskanüle verjüngt.

2. Vorrichtung nach Anspruch 1, **dadurch gekennzeichnet, dass** der Führungsdraht einen maximalen Durchmesser im Bereich von 1,0 bis 1,5 mm, vorzugsweise im Bereich von 1,2 bis 1,3 mm, aufweist.

3. Vorrichtung nach Anspruch 1 oder 2, **dadurch gekennzeichnet, dass** der minimale Innendurchmesser der Punktionskanüle 0,1 bis 0,4 mm größer ist als der maximale Durchmesser des Führungsdrahts.

4. Vorrichtung nach einem der vorangehenden Ansprüche, **dadurch gekennzeichnet, dass** der minimale Innendurchmesser des Führungskatheters 0,05 bis 0,3 mm größer ist als der maximale Außendurchmesser der Punktionskanüle.

5. Vorrichtung nach einem der vorangehenden Ansprüche, **dadurch gekennzeichnet, dass** die Punktionskanüle aus Stahl ist.

6. Vorrichtung nach einem der vorangehenden Ansprüche, **dadurch gekennzeichnet, dass** der Führungskatheter aus Kunststoff ist.

7. Vorrichtung nach einem der vorangehenden Ansprüche, **dadurch gekennzeichnet, dass** der Führungsdraht aus Metall oder Kunststoff ist.

8. Vorrichtung nach einem der vorangehenden Ansprüche, **dadurch gekennzeichnet, dass** am proximalen Ende von Punktionskanüle und Führungskatheter Konnektoren (3, 7) angeordnet sind wobei der Konnektor (3) an der Punktionskanüle in dem Konnektor (7) an dem Führungskatheter so in einer ersten stabilen Einschubposition angeordnet ist, dass die geschliffene Spitze der Punktionskanüle 1 bis 10 mm, besonders bevorzugt 2 bis 6 mm, über das distale Ende des Führungskatheters hinausragt, und wobei durch Ziehen oder Drehen an dem Konnektor (3) der Punktionskanüle diese in eine zweite stabile Einschubposition gebracht wird, bei der die geschliffene Spitze nicht über den Führungskatheter hinausragt.

9. Vorrichtung nach einem der vorangehenden Ansprüche, **dadurch gekennzeichnet, dass** das Ende des Führungsdrahtes, dass im durch die Punktionskanüle hindurchgeführten Zustand über das Ende der Punktionskanüle hinausragt, viertel- bis dreiviertelkreisförmig gebogen ist.

10. Set (12) für die perkutan dilatative Punktionstracheotomie, umfassend eine Vorrichtung nach einem der vorangehenden Ansprüche und einen Dilatator (13) zum Erweitern des Tracheostomas wobei der Dilatator einen Dilatationsbereich mit in Längsrichtung kontinuierlich zunehmendem Durchmesser aufweist und wobei im Dilatator in Längsrichtung ein Führungskanal vorgesehen ist, dessen minimaler Innendurchmesser größer ist als der maximale Durchmesser des Führungsdrahts, sodass der Führungsdraht durch den Führungskanal hindurchführbar ist.

11. Set nach Anspruch 10, **dadurch gekennzeichnet, dass** es zusätzlich einen Prädilatator (19) zum Erweitern des Tracheostomas aufweist, wobei der Prädilatator einen äußeren Durchmesser aufweist, der größer ist als der äußere Durchmesser des Führungskatheters und kleiner als der mittlere Durchmesser des Dilatators (13), und wobei im Prädilatator in Längsrichtung ein Führungskanal vorgesehen ist, dessen minimaler Innendurchmesser größer ist als der maximale Durchmesser des Führungsdrahts, sodass der Führungsdraht durch den Führungskanal hindurchführbar ist, wobei der minimale Innendurchmesser des Führungskanals des Prädilatators 0,1 bis 1,0 mm größer ist als der maximale Durchmesser des Führungsdrahts.

12. Set nach einem der Ansprüche 10 und 11, **dadurch gekennzeichnet, dass** an dem Ende des Führungsdrahtes, das im durch die Punktionskanüle hindurchgeführten Zustand über das Ende der Punktionskanüle hinausragt, eine Einführhilfe (14) angeordnet ist, die in Längsrichtung einen Führungskanal aufweist, dessen minimaler Innendurchmesser größer ist als der maximale Durchmesser des Führungsdrahts, sodass der Führungsdraht durch den Führungskanal hindurchführbar ist, wobei ein Ende des Führungskanals in einem sich konisch verjüngenden Abschnitt der Einführhilfe mündet.

13. Set nach einem der Ansprüche 10-12, **dadurch gekennzeichnet, dass** es zusätzlich ein Skalpell (15), den bzw. einen Prädilatator (19), einen Satz Kompressen (18), einen Armierungskatheter (16), einen Tracheotomietubus, eine oder mehrere Einführhilfen für einen Tracheotomietubus und/oder eine Spritze (17) umfasst.

## Claims

1. Device for percutaneous dilatational tracheostomy, wherein the device has a puncture needle (1), a guide catheter (5) and a guidewire (8), wherein the puncture needle has a ground tip (4) at its distal end, wherein the maximum outer diameter of the puncture needle is smaller than the minimum inner diameter of the guide catheter, and wherein the puncture needle is insertable so far into the guide catheter that the ground tip of the puncture needle projects beyond the distal end of the guide catheter, wherein the maximum diameter of the guidewire is smaller than the minimum inner diameter of the puncture needle, so that the guidewire (8) can be passed through the puncture needle (1) such that its end projects beyond the ground tip (4) of the puncture needle, **characterized in that** the lumen of the puncture needle, at the proximal end of the latter opposite the ground tip, widens in a funnel shape, or a connector (3) whose lumen tapers in a funnel shape in the direction of the lumen of the puncture needle is arranged at the proximal end of the puncture needle, or an adapter (11) whose lumen tapers in a funnel shape in the direction of the lumen of the puncture needle is arranged at the proximal end of the puncture needle.

2. Device according to Claim 1, **characterized in that** the guidewire has a maximum diameter in the range of 1.0 to 1.5 mm, preferably in the range of 1.2 to 1.3 mm.

3. Device according to Claim 1 or 2, **characterized in that** the minimum inner diameter of the puncture needle is 0.1 to 0.4 mm larger than the maximum diameter of the guidewire.

4. Device according to any one of the preceding claims, **characterized in that** the minimum inner diameter of the guide catheter is 0.05 to 0.3 mm larger than the maximum outer diameter of the puncture needle.

5. Device according to any one of the preceding claims, **characterized in that** the puncture needle is made of steel.

6. Device according to any one of the preceding claims, **characterized in that** the guide catheter is made of plastic.

7. Device according to any one of the preceding claims, **characterized in that** the guidewire is made of metal or plastic.

8. Device according to any one of the preceding claims, **characterized in that** connectors (3, 7) are arranged at the proximal end of puncture needle and guide catheter, wherein the connector (3) on the puncture needle is arranged in the connector (7) on the guide catheter in a first stable insertion position such that the ground tip of the puncture needle projects by 1 to 10 mm, particularly preferably by 2 to 6 mm, beyond the distal end of the guide catheter, and wherein, by pulling or turning the connector (3) of the puncture needle, the latter is brought into a second stable insertion position, in which the ground tip does not project beyond the guide catheter.

9. Device according to any one of the preceding claims, **characterized in that** that end of the guidewire projecting beyond the end of the puncture needle, in the state when passed through the puncture needle, has a curved shape from a quarter of a circle to three quarters of a circle.

10. Kit (12) for percutaneous dilatational tracheostomy, comprising a device according to any one of the preceding claims and a dilator (13) for expanding the tracheostoma, wherein the dilator has a dilatation region with a diameter that increases continuously in the longitudinal direction, and wherein a guide channel is provided in the longitudinal direction in the dilator, the minimum inner diameter of which guide channel is larger than the maximum diameter of the guidewire, so that the guidewire can be passed through the guide channel.

11. Kit according to Claim 10, **characterized in that** it additionally has a predilator (19) for expanding the tracheostoma, wherein the predilator has an outer diameter which is larger than the outer diameter of the guide catheter and smaller than the average diameter of the dilator (13), and wherein a guide channel is provided in the longitudinal direction in the predilator, the minimum inner diameter of which guide channel is larger than the maximum diameter of the guidewire, so that the guidewire can be passed through the guide channel, wherein the minimum inner diameter of the guide channel of the predilator is 0.1 to 1.0 mm larger than the maximum diameter of the guidewire.

12. Kit according to either of Claims 10 and 11, **characterized in that** an insertion aid (14) is arranged at that end of the guidewire projecting beyond the end of the puncture needle, in the state when passed through the puncture needle, which insertion aid has, in the longitudinal direction, a guide channel whose minimum inner diameter is larger than the maximum diameter of the guidewire, so that the guidewire can be passed through the guide channel, wherein one end of the guide channel opens into a conically tapering portion of the insertion aid.

13. Kit according to any one of Claims 10-12, **characterized in that** it additionally comprises a scalpel (15), the or a predilator (19), a set of compresses (18), a reinforcing catheter (16), a tracheostomy tube, one or more insertion aids for a tracheostomy tube and/or a syringe (17).

## Revendications

1. Dispositif pour la trachéotomie par dilatation percutanée, le dispositif présentant une canule de ponction (1), un cathéter de guidage (5) et un fil de guidage (8), la canule de ponction présentant une pointe affûtée (4) à son extrémité distale, le diamètre extérieur maximal de la canule de ponction étant inférieur au diamètre intérieur minimal du cathéter de guidage, et la canule de ponction pouvant être insérée dans le cathéter de guidage jusqu'à ce que la pointe affûtée de la canule de ponction fasse saillie au-delà de l'extrémité distale du cathéter de guidage, le diamètre maximal du fil de guidage étant inférieur au diamètre intérieur minimal de la canule de ponction, de telle sorte que le fil de guidage (8) peut être passé à travers la canule de ponction (1) de telle sorte que son extrémité dépasse de la pointe affûtée (4) de la canule de ponction, **caractérisé en ce que** la lumière de la canule de ponction s'élargit en forme d'entonnoir à son extrémité proximale, qui est opposée à la pointe affûtée, ou un connecteur (3) est agencé à l'extrémité proximale de la canule de ponction, dont la lumière se rétrécit en forme d'entonnoir en direction de la lumière de la canule de ponction, ou un adaptateur (11) est agencé à l'extrémité proximale de la canule de ponction, dont la lumière se rétrécit en forme d'entonnoir en direction de la lumière de la canule de ponction.

2. Dispositif selon la revendication 1, **caractérisé en ce que** le fil de guidage présente un diamètre maximal dans la plage de 1,0 à 1,5 mm, de préférence dans la plage de 1,2 à 1,3 mm.

3. Dispositif selon la revendication 1 ou 2, **caractérisé en ce que** le diamètre intérieur minimal de la canule de ponction est supérieur de 0,1 à 0,4 mm au diamètre maximal du fil de guidage.

4. Dispositif selon l'une quelconque des revendications précédentes, **caractérisé en ce que** le diamètre intérieur minimal du cathéter de guidage est supérieur de 0,05 à 0,3 mm au diamètre extérieur maximal de la canule de ponction.

5. Dispositif selon l'une quelconque des revendications précédentes, **caractérisé en ce que** la canule de ponction est en acier.

6. Dispositif selon l'une quelconque des revendications précédentes, **caractérisé en ce que** le cathéter de guidage est en matière plastique.

7. Dispositif selon l'une quelconque des revendications précédentes, **caractérisé en ce que** le fil de guidage est en métal ou en matière plastique.

8. Dispositif selon l'une quelconque des revendications précédentes, **caractérisé en ce que** des connecteurs (3, 7) sont agencés à l'extrémité proximale de la canule de ponction et du cathéter de guidage, le connecteur (3) sur la canule de ponction étant agencé dans le connecteur (7) sur le cathéter de guidage dans une première position d'insertion stable, de telle sorte que la pointe affûtée de la canule de ponction dépasse de 1 à 10 mm, de manière particulièrement préférée de 2 à 6 mm, de l'extrémité distale du cathéter de guidage, et, en tirant ou en tournant le connecteur (3) de la canule de ponction, celle-ci étant amenée dans une deuxième position d'insertion stable, dans laquelle la pointe affûtée ne dépasse pas du cathéter de guidage.

9. Dispositif selon l'une quelconque des revendications précédentes, **caractérisé en ce que** l'extrémité du fil de guidage qui dépasse de l'extrémité de la canule de ponction à l'état passé à travers la canule de ponction est courbée en forme de quart à trois quarts de cercle.

10. Ensemble (12) pour la trachéotomie par dilatation percutanée, comprenant un dispositif selon l'une quelconque des revendications précédentes et un dilatateur (13) pour élargir le trachéostome, le dilatateur présentant une zone de dilatation dont le diamètre augmente de manière continue dans la direction longitudinale et un canal de guidage étant prévu dans le dilatateur dans la direction longitudinale, dont le diamètre intérieur minimal est supérieur au diamètre maximal du fil de guidage, de telle sorte que le fil de guidage peut être passé à travers le canal de guidage.

11. Ensemble selon la revendication 10, **caractérisé en ce qu'**il présente en outre un prédilatateur (19) pour élargir le trachéostome, le prédilatateur présentant un diamètre extérieur qui est supérieur au diamètre extérieur du cathéter de guidage et inférieur au diamètre moyen du dilatateur (13), et un canal de guidage étant prévu dans le prédilatateur dans la direction longitudinale, dont le diamètre intérieur minimal est supérieur au diamètre maximal du fil de guidage, de telle sorte que le fil de guidage peut être guidé à travers le canal de guidage, le diamètre intérieur minimal du canal de guidage du prédilatateur étant supérieur de 0,1 à 1,0 mm au diamètre maximal du fil de guidage.

12. Ensemble selon l'une quelconque des revendications 10 et 11, **caractérisé en ce qu'**à l'extrémité du fil de guidage qui dépasse de l'extrémité de la canule de ponction à l'état passé à travers la canule de ponction, est agencé un auxiliaire d'introduction (14) qui présente dans la direction longitudinale un canal de guidage dont le diamètre intérieur minimal est supérieur au diamètre maximal du fil de guidage, de telle sorte que le fil de guidage peut être passé à travers le canal de guidage, une extrémité du canal de guidage débouchant dans une section de l'auxiliaire d'introduction qui se rétrécit en cône.

13. Kit selon l'une quelconque des revendications 10 à 12, **caractérisé en ce qu'**il comprend en outre un scalpel (15), le ou un prédilatateur (19), un jeu de compresses (18), un cathéter d'armature (16), un tube de trachéotomie, un ou plusieurs auxiliaires d'introduction pour un tube de trachéotomie et/ou une seringue (17).
